# EUROPEAN PATENT SPECIFICATION

(11) **EP 1 779 823 B1**
(45) Date of publication and mention of the grant of the patent: **02.12.2009**
(21) Application number: 05023504.3
(22) Date of filing: 27.10.2005
(51) Int. Cl.: A61F 5/445, A61M 25/10

(54) **Transcecal ileostomy set**
Transcoecalileostomiesansatz
Ensemble d' ileostomy trans-coecale

(43) Date of publication of application: 02.05.2007
(60) Divisional application: 08154030.4
(73) Proprietor: Cedars-Sinai Medical Center, Los Angeles, CA 90048 (US)
(72) Inventor: Ferko, Alexander, 503 31 Vysoka nad Labem (CZ)
(74) Representative: Hutter, Anton

(56) References cited:
- WO-A-01/49224
- WO-A-02/26293
- WO-A-2006/010556
- GB-A- 846 779
- US-A- 5 417 657
- US-B1- 6 461 327

## Description

This invention relates generally to medical devices. More particularly, the field of art into which this invention falls is ileostomy sets used for drainage and collection of feces straight from the ileum (i.e. the large intestine is bypassed) through the abdominal wall.

Defunctioning surgical loop ileostomy is a standard technique used for temporary diversion for left-sided colon and rectum resections in high-risk patients. When ileostomy is unnecessary anymore, it is taken down. A patient shall undergo another surgery - laparotomy, during which a loop ileostomy is closed. Even such an operation is associated with mortality and rather high morbidity.

The submitted invention is based on a special balloon catheter, which allows total drainage of intestinal fluids/feces from the small intestine such that the colon after a resection is bypassed. A special bag for collection of feces is connected to the balloon catheter outside of the human body.

WO 01/49224 discloses a continent ostomy port device comprising a catheter portion with a proximal bolster to seals and anchor the device and a dutal chamber.

This submitted transcecal ileostomy set has been constructed with an intention to close up the ileostomy without surgery, i.e. to spare a patient one operation. The solution of the present invention allows, upon removal of the balloon catheter, to close the ileostomy by means of a bioresorbable loop that purses the large intestine wall percutaneously.

In addition, the balloon catheter of the present invention is equipped with one more inflatable balloon; upon insertion of the balloon catheter into the desired place, the balloon is inflated inside the large intestine which prevents the balloon catheter from undesirable movement.
The present disclosure relates to a transcecal ileostomy as claimed hereafter.
Fig. 1 is a cross-section side view of the preferred embodiment of the complete transcecal ileostomy set (without the bag for feces that is not a part of the invention).
Fig. 2 is a plan view of the plastic holder from the Fig. 1.
Fig. 3 is a cross-section side view of the plastic holder from the Figs. 1.

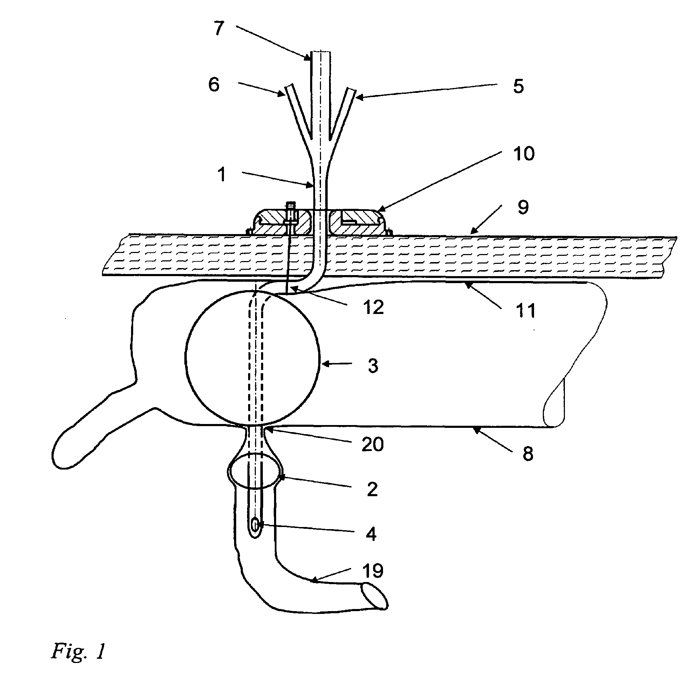

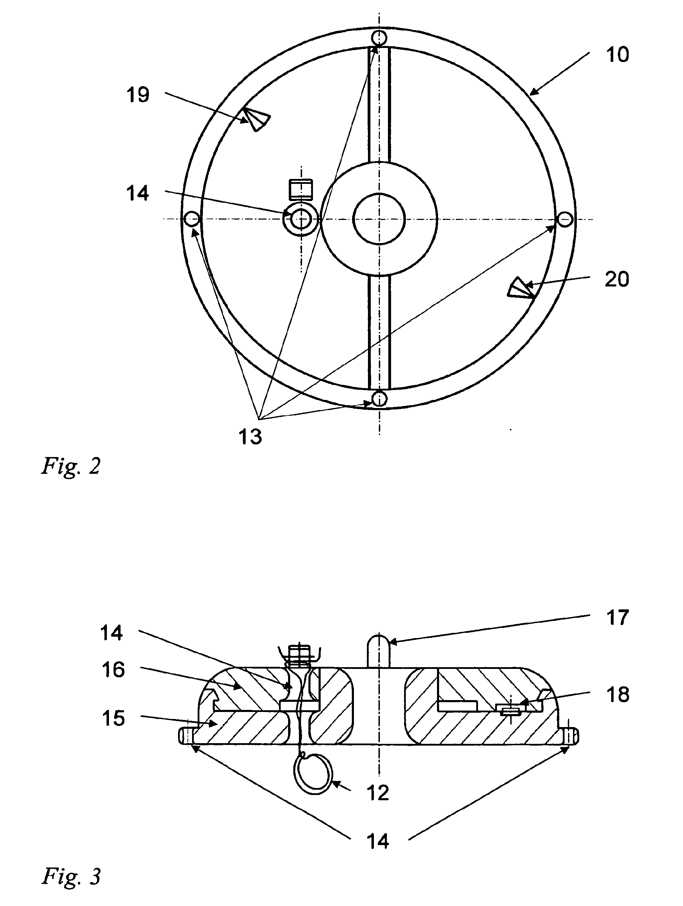

The Fig. 1 shows the transcecal ileostomy set of the present invention. It consists of a balloon catheter 1, equipped with two balloons - blocking balloon 2 and fixation balloon 3 - in a distal portion thereof, with perforation 4 at the very distal end. The balloon catheter 1 is divided into three lumens - one is equipped with a blocking balloon valve 5 at a proximal end, one with fixation balloon valve 6 at a proximal end and the last one is ended with intestinal fluid outlet 7 at its proximal end. The balloon catheter 1 is fastened against movement inside the large intestine 8 by means of the inflated fixation balloon 3 and out of a patient's body on the surface of an abdominal wall 9 by means of a plastic holder 10. In addition to that, the balloon catheter 1 is fastened to a large intestine wall 11 by using a bioresorbable loop 12. That said bioresorbable loop 12 passes through the large intestine wall 11 as well as abdominal wall 9 and is tied up to the plastic holder 10 (as shown in the Fig. 3).

The said plastic holder 10 is fixed to the outer side of the abdominal wall 9 by using fixing apertures 13 as best-depicted in the Fig. 2; it serves for holding of both balloon catheter 1 and bioresorbable loop 12. The bioresorbable loop 12 passes from the site of incision into the large intestine wall 11 (through which the balloon catheter 1 is inserted) through the abdominal wall 9 and the aperture 14 of both stable 15 and rotary 16 parts of the plastic holder 10 and is tied up thereon (as shown in the Fig. 3). The rotary part 16 of the plastic holder 10 is affixed with protrusion 17 serving as a block when tightening the bioresorbable loop 12. To tighten the said bioresorbable loop 12, the rotary part 16 is turned clockwise; an anticlockwise movement of is blocked by a system of toothing with latch 18.

In a preferred embodiment according to the present invention, the balloon catheter 1 is introduced through a small incision into the large intestine wall 11 such that its distal portion with the fixation balloon 3 is inserted into the large intestine 8 and the blocking balloon 2 into the small intestine 19 behind the Bauhin valve 20 (as shown in the Fig. 1).

The bioresorbable loop 12, e.g. monofilamentous surgical suture PDS (absorbable polydioxanone surgical suture) in this embodiment, is specially placed around the balloon catheter 1 in the shape of purse-string suture to seal space between the balloon catheter 1 and the incision edges on the large intestine wall 11. Then both ends of the bioresorbable loop 12 are threaded through the abdominal wall 9 such to form a lasso-loop (as shown in the Fig. 3). This lasso-loop holds the large intestine wall 11 tight to the abdominal wall 9 and allows a closure of the incision after removal of the balloon catheter 1. The proximal end of the balloon catheter 1 is held by the plastic holder 10 being fixed to the body surface as shown in the Fig. 1.

A syringe is put on the fixation balloon valve 6 to inflate the fixation balloon 3 to fix the inserted balloon catheter 1 in situ. Then a syringe is put on the blocking balloon valve 5 to inflate the blocking balloon 2 to completely obstruct the small intestine 19. The intestinal fluid is thereby forced to flow through the perforation 4 of the balloon catheter 1 and therethrough up to an intestinal fluid outlet 7 at the proximal end of the balloon catheter 1 (that is connected to a common ileostomic bag that is not a part of this invention).

When this device is unnecessary anymore and the balloon catheter 1 is to be removed, both blocking balloon 2 and fixation balloon 3 are deflated first by using a syringe again. The balloon catheter 1 is then removed from a patient's body by simple pulling.

To avoid an intestinal fluid leakage through the incision when the balloon catheter 1 is removed, the large intestine wall 11 shall be closed. Turning the rotary part 16 of the plastic holder 10 clockwise from the default position 19 to the position of tightened loop 20 (as shown in the Fig. 2), the bioresorbable loop 12 purses the large intestine wall 11 until completely closed.

When closed, the said large intestine wall 11 gets healed in several days; the plastic holder 10 is removed. The bioresorbable loop 12 gets resorbed later on in situ thus no further intervention is needed.

## Claims

1. A transcecal ileostomy set designed for drainage of intestinal fluids from the small intestine (19) comprising a balloon catheter (1 ) that is percutaneously introduced through an abdominal wall (9) and a large intestine wall (11) into the small intestine (19) is **characterized by** a fixation balloon (3) affixed to a distal portion of the balloon catheter (1) such that it is inflated in the large intestine (8) to fill up its entire lumen, and a blocking balloon (2) affixed to a distal portion of the balloon catheter (1) such that it is inflated in the small intestine (19) behind a Bauhin valve (20) to obstruct its entire lumen.

2. The transcecal ileostomy set according to claim 1, **characterized in that** the balloon catheter (1) comprises three lumens of which one is equipped with a fixation balloon valve (6) at its proximal end serving for inflation of the fixation balloon (3), one is equipped with a blocking balloon valve (5) at its proximal end serving for inflation of the blocking balloon (2), and one lumen with an intestinal fluid outlet (7) at its proximal end serving for drainage of the intestinal fluids.

## Patentansprüche

1. Gerät für die transzäkale Ileostomie, das für die Drainage von Darmfluiden aus dem Dünndarm (19) konzipiert ist, umfassend einen Ballonkatheter (1), der perkutan durch eine Bauchwand (9) und eine Wand des Dickdarms (11) in den Dünndarm (19) eingeführt wird, ist **gekennzeichnet durch** einen Fixierballon (3), der an einem distalen Teil des Ballonkatheters (1) derart befestigt ist, dass er im Dickdarm (8) aufgeblasen wird, um sein gesamtes Lumen zu füllen, und **durch** einen Blockierballon (2), der an einem distalen Teil des Ballonkatheters (1) derart befestigt ist, dass er im Dünndarm (19) hinter einem Bauhin-Ventil (20) aufgeblasen wird, um sein gesamtes Lumen zu blockieren.

2. Gerät für die transzäkale Ileostomie nach Anspruch 1, **dadurch gekennzeichnet, dass** der Ballonkatheter (1) drei Lumen umfasst, von denen eines mit einem Fixierballonventil (6) an seinem proximalen Ende, das dem Aufblasen des Fixierballons (3) dient, ausgestattet ist, eines mit einem Blockierballonventil (5) an seinem proximalen Ende, das dem Aufblasen des Blockierballons (2) dient, ausgestattet ist, und ein Lumen mit einem Darmfluidauslass (7) an seinem proximalen Ende der Drainage des Darmfluids dient.

## Revendications

1. Dispositif d'iléostomie transcæcale conçu pour le drainage de liquides intestinaux de l'intestin grêle (19), comprenant un cathéter à ballonnet (1) qui est introduit dans l'intestin grêle (19) par voie percutanée au travers d'une paroi abdominale (9) et d'une paroi du gros intestin (11) et **caractérisé par** un ballonnet de fixation (3) qui est attaché à une portion distale du cathéter à ballonnet (1) de manière à être déployé dans le gros intestin (8) pour remplir la totalité de la lumière et par un ballonnet de blocage (2) qui est attaché à une portion distale du cathéter à ballonnet (1) de manière à être gonflé dans l'intestin grêle (19) en amont d'une valvule de Bauhin (20) pour obstruer la totalité de la lumière.

2. Dispositif d'iléostomie transcæcale selon la revendication 1, **caractérisé en ce que** le cathéter à ballonnet (1) comprend trois lumières dont une est équipée à son extrémité proximale d'une valve du ballonnet de fixation (6) qui sert au gonflement du ballonnet de fixation (3), une est équipée à son extrémité proximale d'une valve du ballonnet de blocage (5) qui sert au gonflement du ballonnet de blocage (2) et une est équipée à son extrémité proximale d'un orifice de sortie du liquide intestinal (7) qui sert au drainage des liquides intestinaux.
